# EUROPEAN PATENT SPECIFICATION

(11) **EP 1 202 058 B1**
(45) Date of publication and mention of the grant of the patent: **14.05.2008**
(21) Application number: 01926009.0
(22) Date of filing: 27.04.2001
(51) Int. Cl.: G01N 33/543

(54) **CHROMATOGRAPHY MEASURING INSTRUMENT**
MESSINSTRUMENT FÜR CHROMATOGRAPHIE
INSTRUMENT DE MESURE PAR CHROMATOGRAPHIE

(30) Priority: 28.04.2000 JP 2000130458
(43) Date of publication of application: 02.05.2002
(73) Proprietor: MATSUSHITA ELECTRIC INDUSTRIAL CO., LTD, Kadoma-shi, Osaka 571-8501 (JP)
(72) Inventor: TAKAHASHI, Mie, Niihama-shi, Ehime 792-0026 (JP); NADAOKA, Masataka, Iyo-shi, Ehime 799-3113 (JP); TANAKA, Hirotaka, Matsuyama-shi, Ehime 791-1102 (JP); KITAWAKI, Fumihisa, Kadoma-shi Osaka 571-0064 (JP)
(74) Representative: Balsters, Robert
(86) International application number: PCT/JP2001/003683
(87) International publication number: WO 2001/084151

(56) References cited:
- EP-A- 1 104 886
- WO-A-95/16207
- WO-A-98/32018
- JP-A- 10 332 700
- JP-A- 11 044 689
- JP-A- 11 094 817

## Description

### TECHNICAL FIELD

The present invention relates to a chromatography , measuring device for qualitatively or quantitatively measuring substance to be tested.

### BACKGROUND ART

Conventionally, a chromatography measurement which utilizes an antigen-antibody reaction or the like is generally used as a method for implementing a chemical test or a clinical test for liquid samples, such as examination of water and urinalysis. This chromatography measurement is a method for separating a mixture according to its components. A chromatography measuring device employed for the chromatography measurement comprises a sample application part to which a liquid sample is usually applied, a marker reagent holding part in which a marker reagent which can be moved by permeation of the liquid sample is held, a specific protein immobilization part in which a protein that is specifically bonded to an analyte in a liquid sample which flows in the specific protein immobilization part with the marker reagent is immobilized, a reactive layer in which a specific binding reaction is caused, and a water-absorbing part for absorbing the sample which flows therein; these constituent members are sequentially laminated or connectively aligned on a support body composed of plastic or the like.

Next, a chromatography measurement employing such typical chromatography measuring device will be described.

A liquid sample is applied to the sample application part, and then it reaches the region of the marker reagent holding part. Then, a marker reagent held in the region of the marker reagent holding part is dissolved due to the permeation of the liquid sample and permeates the region of the reactive layer with the liquid sample. On the region of the reactive layer, there is the specific protein immobilization part, and when the liquid sample includes an analyte therein, a specific protein immobilized in the specific protein immobilization part performs a binding reaction, which entails coloration or coloring, with a complex of the analyte and the marker reagent, the judgement can be made by a visual detecting method. On the other hand, when the liquid sample does not include an analyte therein, no binding reaction is caused and neither coloration nor coloring is seen. The liquid sample is finally absorbed into the water-absorbing part provided in a bottom region of the chromatography measuring device, and the reaction is ended.

As described above, since the chromatography measurement using the conventional chromatography measuring device quite easily judges the result of the test, it is widely applicable and can be utilized for test of various analytes.

However, in the chromatography measurement using the above-described conventional chromatography measuring device, moisture in a liquid sample applied at chromatographic development is naturally evaporated from the top surface and side faces of the chromatography measuring device, whereby an amount of an analyte which flows into a measurement region is ununiform and amounts of a liquid sample and marker reagent flowing on the chromatography measuring device for a definite period of time are not constant, and therefore it is impossible to perform an accurate chromatography measurement.

Further, since the conventional chromatography measuring device is manufactured by cutting a sheet into the specimen size, in which the constituent members such as the sample addition part, the marker reagent holding part, the specific protein immobilization part, the reactive layer, and the water-absorbing part are laminated or connected on the support body, the members laminated or connected on the support body are exfoliated when the sheet is cut into the specimen size.

Further, for performing an accurate chromatography measurement employing the conventional chromatography measuring device, the above-described constituent members of the sample addition party the marker reagent holding part, the specific protein immobilization part, the reactive layer, and the water-absorbing part are thought to be indispensable and impossible to be eliminated. Thus, manufacture of the chromatography measuring device requires a certain cost, resulting in cost overrun.

The present invention is made to solve the above-mentioned problems and has for its object to provide a low-cost chromatography measuring device having high sensitivity and high performance, whose constituent members are to be reduced and whose specimen manufacturing process is simplified.

EP-A-1 104 886, which is prior art under Art. 54(3) and (4) EPC, discloses a chromatography specimen which is covered with a liquid-impermeable sheet (a fluid impermeable barrier), wherein only the upstream end part of a chromatography specimen, that is, a sample addition part, has an opening part.

JP-A-11 094 817 discloses a test strip which is covered with a liquid non-permeable sheet, wherein an opening part is formed in the sheet only in the sample addition part, namely in the end region on chromatographic upstream.

JP-A-10 332 700 discloses a chromatography test strip, wherein an air flowing clearance is formed between a member for adding sample and a member for absorbing the sample to be tested of the chromatographic test strip, that is in the middle region of the chromatography specimen.

EP-A-1 003 038 & JP-A-11 044 689 discloses a test strip having an opening part in a color developing region which is provided in a sample addition part and in the middle region of the chromatography specimen.

WO-A-95/16207 discloses a chromatography specimen which is covered with a liquid-impermeable sheet (a fluid impermeable barrier), wherein an aperture for adding a sample is provided in the center of the fluid impermeable barrier.

### DISCLOSURE OF THE INVENTION

According to the present invention (Claim 1), there is provided a chromatography measuring device which has a chromatography specimen as a specimen for performing a chromatography measurement and qualitatively or quantitatively measures substance to be tested, which is applied to the chromatography specimen, wherein the chromatography specimen is open over at least the top and side surfaces of both end regions on chromatographic upstream and downstream, and is adherently covered with a liquid-impermeable sheet material at all parts of the top surface except for both of its end regions on chromatographic upstream and downstream.

In the so-constituted chromatography measuring device, an applied liquid sample permeates toward the chromatography downstream by a capillary phenomenon, moisture evaporation does not occur in the region adherently covered with the liquid-impermeable sheet material, and the liquid sample reaches the open part, and then moisture evaporation is progressed and the sample is dried. On the other hand, the liquid sample is held in a sample application part, so that the liquid sample does not flow back but is developed in the chromatographic downstream direction. Therefore, it is possible to realize a low-cost chromatography measuring device with sensitivity and performance equal to or higher than those in prior arts, in which the permeation direction and condition of the liquid sample are made uniform, and the liquid sample and a marker reagent flowing for a definite period of time can flow at uniform concentrations, and a water-absorbing part for absorbing the sample need not be provided in the downstream region, which results in reduction of constituent members and simplification of a specimen manufacturing process.

Further, since the top surface of the chromatography measuring device is covered with the liquid-impermeable sheet material, there is no need to wrap the liquid-impermeable sheet material around the chromatography specimen to its back side at the manufacture of the chromatography measuring device, and there is no need to worry about exfoliation of the members at cutting when the manufactured chromatography sheet is cut into the chromatography specimen size, since the surface of the chromatography sheet is covered adherently with the liquid-impermeable sheet material. Therefore, there is no need to provide a water-absorbing part for absorbing the sample in the downstream region, resulting in a low-cost chromatography measuring device with sensitivity and performance equal to or higher than those in prior arts, in which the constituent members are reduced and a specimen manufacturing process is simplified.

According to Claim 2 of the present invention, in the chromatography measuring device as defined in Claim 1, the side surfaces of the chromatography specimen, except for both of its end regions on chromatographic upstream and downstream, are further adherently covered with the liquid-impermeable sheet material.

In the so-constituted chromatography, measuring device, an applied liquid sample permeates toward the chromatography downstream by a capillary phenomenon, moisture evaporation does not occur in the region adherently covered with the liquid-impermeable sheet material, and the liquid sample reaches the open part, and then moisture evaporation is progressed and the sample is dried. On the other hand, the liquid sample is held in a sample application part, so that the liquid sample does not flow back but is developed in the chromatographic downstream direction. Therefore, there is no need to provide a water-absorbing part for absorbing the sample in the downstream region, resulting in a low-cost chromatography measuring device with sensitivity and performance equal to or higher than those in prior arts, in which the constituent members are reduced and a specimen manufacturing process is simplified.

According to Claim 3, of the present invention, in the chromatography measuring device as defined in Claim 1, the side surfaces, and bottom surface of the chromatography specimen, except for both of its end regions on chromatographic upstream and downstream, are further adherently covered with the liquid-impermeable sheet material.

In the so-constituted chromatography measuring device, an applied liquid sample permeates toward chromatography downstream by a capillary phenomenon, moisture evaporation does not occur in the region adherently covered with the liquid-impermeable sheet material, and the liquid sample reaches the open part, and then moisture evaporation is progressed and the sample is dried. On the other hand, the liquid sample is held in a sample application part, so that the liquid sample does not flow back but is developed in the chromatographic downstream direction. Further, the liquid-impermeable sheet material also serves as a reactive layer support body, and thus the number of constituents for constituting the chromatography measuring device can be reduced. Therefore, there is no need to provide a water-absorbing part for absorbing the sample in the downstream region, resulting in a low-cost chromatography .. measuring device with sensitivity and performance equal to or higher than those in prior arts, in which the constituent members are reduced and a specimen manufacturing process is simplified.

According to Claim 4 of the present invention, in the chromatography measuring device as defined in any of Claims 1 to 3, a measurement region at least from a marker reagent holding part in which a marker reagent is held, located upstream, to a specific protein immobilization part in which a specific protein is immobilized, located downstream, in the chromatography specimen is adherently covered with the liquid-impermeable sheet material.

In the so-constituted chromatography measuring device an applied liquid sample reacts and simultaneously permeates toward the chromatography downstream by a capillary phenomenon between the reagent holding region and the specific protein holding region, moisture evaporation does not occur in the region adherently covered with the liquid-impermeable sheet material, and the liquid sample reaches the open part, and then moisture evaporation is progressed and the sample is dried. On the other hand, the liquid sample is held in a sample application part, so that the liquid sample does not flow back but is developed in the chromatographic downstream direction. Therefore, there is no need to provide a water-absorbing part for absorbing the sample in the downstream region, resulting in a low-cost chromatography measuring device with sensitivity and performance equal to or higher than those in prior arts, in which the constituent members are reduced and a specimen manufacturing process is simplified.

According to Claim 5 of the present invention, in the chromatography measuring device as defined in any of Claims 1 to 4, the chromatography specimen is constituted by laminating or connecting plural porous materials.

In the so-constituted chromatography measuring device, an applied liquid sample permeates toward the chromatography downstream by a capillary phenomenon, moisture evaporation does not occur in the region adherently covered with the liquid-impermeable sheet material, and the liquid sample reaches the open part, and then moisture evaporation is progressed and the sample is dried On the other hand, the liquid sample is held in a sample application part, so that the liquid sample does hot flow back but is developed in the chromatographic downstream direction. Therefore, there is no need to provide a water-absorbing part for absorbing the sample in the downstream region, resulting in a low-cost chromatography measuring device with sensitivity and performance equal to or higher than those in prior arts, in which the constituent members are reduced and a specimen manufacturing process is simplified.

According to Claim 6 of the present invention, in the chromatography measuring device as defined in any of Claims 1 to 4, the chromatography specimen is composed of a single-layer porous material.

In the so-constituted chromatography measuring device, an applied liquid sample permeates toward the chromatography downstream by a capillary phenomenon, moisture evaporation does not occur in the region adherently covered with the liquid-impermeable sheet material, and the liquid sample reaches the open part, and then moisture evaporation is progressed and the sample is dried. On the other hand, the liquid sample is held in a sample application part , so that the liquid sample does not flow back but is developed in the chromatographic downstream direction. Therefore, there is no need to provide a water-absorting part for absorbing the sample in the downstream region. Further, since a basic member for constituting the chromatography specimen is a single layer it is possible to realize a low-cost chromatography measuring device with sensitivity and performance equal to or higher than those in prior arts, in which the constituent members are reduced and a specimen manufacturing process is simplified.

According to Claim 7 of the present invention, in the chromatography measuring device as defined in Claim 6, the single-layer porous material is nitrocellulose.

In the so-constituted chromatography measuring device, only a minute amount of liquid application is sufficient, and an applied liquid sample permeates toward the chromatography downstream by a capillary phenomenon, moisture evaporation does not occur in the region adherently covered with the liquid-impermeable sheet material, and the liquid sample reaches the open part, and then moisture evaporation is progressed and the sample is dried. On the other hand, the liquid sample is held in a sample application part, so that the liquid sample does not flow back but is more effectively developed in the chromatographic downstream direction. Therefore, there is no need to provide a water-absorbing part for absorbing the sample, which has been required, in the downstream region. Further, since a basic member for constituting the chromatography specimen is a single layer it is possible to realize a low-cost chromatography measuring device with sensitivity and performance equal to or higher than those in prior arts, in which the constituent members-are reduced and a specimen manufacturing process is simplified.

According to Claim 8 of the present invention, in the chromatography measuring device as defined in any of Claims 1 to 7, the chromatographic downstream region which is not covered with the liquid-impermeable sheet material is covered with a gas-permeable material.

In the so-constituted chromatography measuring device, an applied liquid sample permeates toward the chromatography downstream by a capillary phenomenon, moisture evaporation does not occur in the region adherently, covered with the liquid-impermeable sheet material, and when the liquid sample reaches the downstream region covered with the gas-permeable material, moisture evaporation is progressed and the sample is dried. On the other hand, the liquid sample is held in a sample application part, so that the liquid sample does not flow back but is developed in the chromatographic downstream direction. Further, since the downstream region is covered with the gas-permeable material, an exposed reactive layer can not be touched directly with one's hand, so that the reactive layer is not polluted by substance attached to the hand, thereby keeping water absorption. Even when the downstream region of the specimen is touched with one's hand, the liquid sample is not attached to the hand. Therefore, there is no need to provide a water-absorbing part for absorbing the sample in the downstream region, resulting in a low-cost chromatography measuring device with sensitivity and performance equal to or higher than those in prior arts, which has its constituent members reduced and a specimen manufacturing process simplified, and is usable more safely and sanitarily.

According to Claim 9 of the present invention, in the chromatography measuring device as defined in Claim 8, the gas-permeable material is an arbitrary porous thin-film material such as a nonwoven fabric.

In the so-constituted chromatography measuring device, due to the thin-film material with excellent gas permeability, its own absorbency is low, and when a liquid sample reaches the downstream region, moisture evaporation is progressed and the sample can be dried, and further since the downstream region is covered with the gas-permeable material, an exposed reactive layer can not be touched directly with one's hand, so that the reactive layer is not polluted by substance attached to the hand, thereby keeping water absorption. Even when the downstream region of the specimen is touched with one's hand, the liquid sample is not attached to the hand. Therefore, there is no need to provide a water-absorbing part for absorbing the sample in the downstream region, resulting in a low-cost chromatography measuring device with sensitivity and performance equal to or higher than those in prior arts, which has its constituent members, reduced .and a specimen manufacturing process simplified, and, is usable more safely and sanitarily.

According to Claim 10 of the present invention, in the chromatography measuring device as defined in Claim 8, the gas-permeable material is retiform tissue.

In the so-constituted chromatography measuring device, due to retiform tissue, it is low in absorbency itself while excellent in gas permeability, and when a liquid sample reaches the downstream region, moisture evaporation is progressed and the sample can be dried, and further since the downstream region is covered with the gas-permeable material, an exposed reactive layer can not be touched directly with one's hand, so that the reactive layer is not polluted by substance attached to the hand, thereby keeping water absorption. Even when the downstream region of the specimen is touched with one's hand, the liquid sample is not attached to the hand. Therefore, there is no need to provide a water-absorbing part for absorbing the sample in the downstream region, resulting in a low-cost chromatography measuring device with sensitivity and performance equal to or higher than those in prior arts, which has its constituent members reduced and a specimen manufacturing process simplified, and usable more safely and sanitarily.

According to claim 11 of the present invention, in the chromatography , measuring device as defined in any of Claims 1 to 7, a space forming part for forming arbitrary space is provided on the chromatographic downstream region which is not covered with the liquid-impermeable sheet material.

In the so-constituted chromatography measuring device, since the space forming part is provided in the downstream region, an exposed reactive layer can not be touched directly with one's, hand, so that the reactive layer is not polluted by substance attached to the hand, thereby keeping water absorption. Even when the downstream region of the specimen is touched with one's hand, a liquid sample is not attached to the hand. Further, since space is formed in the downstream region, the liquid sample reaches the downstream region and then moisture evaporation is progressed and the sample can be dried. Therefore, there is no need to provide a water-absorbing part for absorbing the sample in the downstream region, resulting in a low-cost chromatography measuring device with sensitivity and performance equal to or higher than those in prior arts, which has its constituent members reduced and a specimen manufacturing process simplified, and is usable more safely and sanitarily.

According to claim 12 of the present invention, in the chromatography measuring device as defined in claim 11, a gap part is provided in an arbitrary region such as at the end or on a parallels side of the chromatograhic downstream region in the space forming part, or on the top surface of the space forming part, so as to enable air inflow.

In the so-constituted chromatography measuring device, since the space forming part is provided in the downstream region, an exposed reactive layer can not be touched directly with one's hand, so that the reactive layer is not polluted by substance attached to the hand, thereby keeping water absorption. Even when the downstream region of the specimen is touched with one's hand, a liquid sample is not attached to the hand. Further, since space is formed in the downstream region and the gap part is provided at an arbitrary position, resulting in good gas permeability, and the liquid sample reaches the downstream region and then moisture evaporation is progressed and the sample can be dried. Therefore, there is no need to provide a water-absorbing part for absorbing the sample in the downstream region, resulting in a low-cost chromatography measuring device with sensitivity and performance equal to or higher than those in prior arts, which has its constituent members reduced and a specimen manufacturing process simplified, and is usable more safely and sanitarily.

According to Claim 13 of the present invention, in the chromatography measuring device as defined in Claim 11 or 12, the space forming part is composed of a liquid-impermeable material.

In the so-constituted chromatography measuring device, since the space forming part is provided in the downstream region employing the liquid-impermeable material, an exposed reactive layer is not touched directly with one's hand, so that the reactive layer is not polluted by substance attached to the hand, thereby keeping water absorption. Even when the downstream region of the specimen is touched with one's hand, a liquid sample is not attached to the hand. Further, since space is formed in the downstream region, the liquid sample reaches the downstream region and then moisture evaporation is progressed and the sample can be dried. Therefore, there is no need to provide a water-absorbing part for absorbing the sample in the downstream region, resulting in a low-cost chromatography measuring device with sensitivity and performance equal to or higher than those in prior arts, which has its constituent members reduced and a specimen manufacturing process simplified, and is usable more safely and sanitarily.

According to Claim 14 of the present invention, in the chromatography measuring device as defined in any of Claims 1 to 13, the chromatography specimen is an immunochromatography specimen employing an antigen-antibody reaction.

In the so-constituted chromatography measuring device, it is possible to realize a low-cost chromatography measuring device with sensitivity and performance equal to or higher than those in prior arts, which can measure many measuring objects by obtaining an antibody or antigen for the measuring objects and can be used more safely and sanitarily.

Further, the immunochromatography specimen described here indicates a specimen for detecting substance to be tested in the sample liquid employing an antigen-antibody reaction on a support body where chromatographic development occurs.

According to Claim 15 of the present invention, in the chromatography measuring device as defined in any of Claims 1 to 14, the chromatography specimen is a dry analysis element.

In the so-constituted chromatography measuring device, it is possible to minimize denaturation of a protein or the like and to store the measuring device for a long term.

According to Claim 16 of the present invention, in the chromatography measuring device as defined in any of Claims 1 to 15, the chromatography specimen is a one-step specimen.

In the so-constituted chromatography measuring device, a pretreatment of a sample liquid is not required and it is only required to apply the sample liquid to the specimen, thereby simplifying an operation.

### BRIEF DESCRIPTION OF DRAWINGS

Figure 1 is a diagram illustrating a structure of a chromatography measuring device according to a first embodiment of the present invention.

Figure 2 is a diagram illustrating a structure of a chromatography specimen without a liquid-impermeable sheet material of the chromatography measuring device according to the first embodiment of the present invention.

Figure 3 is a diagram illustrating a structure of a chromatography measuring device according to a second embodiment of the present invention.

Figure 4 is a diagram illustrating a structure of a chromatography specimen without a liquid-impermeable sheet material of the chromatography measuring device according to the second embodiment of the present invention.

Figure 5 is a sectional view of a chromatography measuring device according to a third embodiment of the present invention, which is cut parallel to the chromatographic direction.

Figure 6 is a sectional view of the chromatography measuring device according to the third embodiment of the present invention, which is cut perpendicular to the chromatographic direction.

Figure 7 is a sectional view of a chromatography measuring device according to a fourth embodiment of the present invention, which is cut parallel to the chromatographic direction.

Figure 8 is a sectional view of the chromatography measuring device according to the fourth embodiment of the present invention, which is cut perpendicular.to the chromatographic direction.

Figure 9 is a diagram illustrating a structure of a chromatography measuring device according to a fifth embodiment of the present invention.

Figure 10 is a sectional view of the chromatography measuring device according to the fifth embodiment of the present invention, which is cut parallel to the chromatographic direction.

Figure 11 is a sectional view of the chromatography measuring device according to the fifth embodiment of the present invention, which is cut perpendicular to the chromatographic direction.

Figure 12 is a diagram illustrating a structure of a chromatography measuring device according to a sixth embodiment of the present invention.

Figure 13 is a diagram illustrating a structure of a chromatography measuring device according to a seventh embodiment of the present invention.

Figure 14 is a sectional view of the chromatography measuring device according to the seventh embodiment of the present invention, which is cut perpendicular to the chromatographic direction.

Figure 15 is a sectional view of the chromatography measuring device according to the seventh embodiment of the present invention, which is cut perpendicular to the chromatographic direction.

Figure 16 is a diagram illustrating a structure of the chromatography measuring device according to the seventh embodiment of the present invention.

Figure 17 is a diagram illustrating a structure of the chromatography measuring device according to the seventh embodiment of the present invention.

Figure 18 is a sectional view of the chromatography measuring device according to the seventh embodiment of the present invention, which is cut parallel to the chromatographic direction.

### BEST MODE TO EXECUTE THE INVENTION

Hereinafter, embodiments according to the present invention will be described with reference to the figures. The embodiments described here are given only as examples and the present invention is not restricted to these embodiments.

### (Embodiment 1)

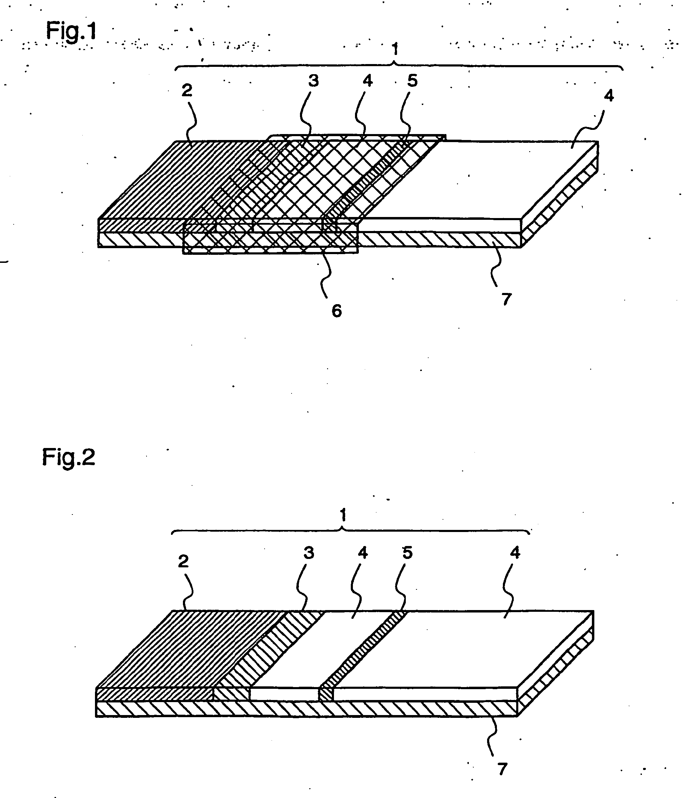

Hereinafter, a chromatography measuring device according to a first embodiment of the present invention will be described with reference to figures 1 and 2.

Figure 1 is a diagram illustrating the chromatography measuring device according to the first embodiment of the present invention and figure 2 illustrates a chromatography specimen without a liquid-impermeable sheet material of the chromatography measuring device according to the first embodiment.

Figure 1 illustrates the chromatography measuring device for qualitatively or quantitatively measuring substance to be tested, which is applied to a chromatography specimen 1, and the chromatography specimen 1 is adherently covered with a liquid-impermeable sheet material 6 except for both of its end regions on chromatographic upstream and downstream.

The chromatography specimen 1 shown in figure 2 comprises a sample application part 2 composed of a porous material such as a nonwoven fabric having high water absorption, to which a liquid sample is added or applied, a marker reagent holding part 3 in which a marker reagent which can be moved by permeation of the liquid sample is held, a reactive layer 4 composed of a porous material such as nitrocellulose, in which a specific binding reaction is performed with an analyte in the liquid sample which flows into the reactive layer 4, and a specific protein immobilization part 5 in which a protein that is specifically bounded to the analyte in the liquid sample which flows onto the region of the reactive layer 4 is immobilized.

Further, this chromatography specimen 1 is constituted by laminating or connecting both regions of the sample application part 2 and reactive layer 4 composed of a porous material on a support body 7 composed of plastic or the like, and the marker reagent holding part 3 and the specific protein immobilization part 5 are provided on the members composed of the porous material. In the present invention, lamination indicates a state where plural different materials are more or less overlapped with adjacent members, and connection indicates a state where plural different materials are not overlapped but adherent to adjacent members.

Further, in the chromatography measuring device 1 according to the present invention, the chromatography specimen 1 shown in figure 1 is adherently covered with a liquid-impermeable sheet material 6 made of plastic tape or the like except for both of its end regions on chromatographic upstream and downstream.

While in the present invention an arbitrary surface favorably indicates a state where the two regions upstream and downstream in the chromatography specimen 1 are equal in size or the downstream region is broader, states other than this are also available.

Next, a chromatography measurement employing the chromatography measuring device (See figure 1) according to the first embodiment of the present invention will be described.

In the chromatography measuring device shown in figure 1, a liquid sample is applied to the sample application part 2, and then it reaches the region of the marker reagent holding part 3. Then, a marker reagent held in the region of the marker reagent holding part 3 is dissolved due to the permeation of the liquid sample and permeates the region of the reactive layer 4 with the liquid sample. On the region of the reactive layer 4, there is the specific protein immobilization part 5, and when the liquid sample includes an analyte therein, a specific protein immobilized in the specific protein immobilization part 5 performs a binding reaction with a complex of the analyte and the marker reagent, and a color reaction is seen in the region of the specific protein immobilization part 5. On the other hand, when the liquid sample does not include an analyte therein, no binding reaction is caused and no color reaction is seen. The liquid sample finally permeates a bottom region of the chromatography specimen 1, and the reaction is ended.

At this time, a measurement region at least from the marker reagent holding part 3 located upstream to the specific protein immobilization part 5 located downstream on the surface of the chromatography specimen 1 is adherently covered with the liquid-impermeable sheet material 6 made of plastic tape or the like, thereby preventing evaporation of moisture in the measurement region, making the amount of the liquid sample permeating the measurement area uniform, and further making concentrations of the liquid sample and marker reagent flowing in the measurement region for a definite period of time constant, resulting in an accurate.chromatography measurement.

Further, the downstream region of the chromatography specimen 1 is not covered with the liquid-impermeable sheet material 6, whereby moisture is gradually evaporated.as the liquid sample permeates a bottom open part as a region where the bottom of the chromatography specimen 1 is opened. Since an evaporation rate of the liquid sample in the bottom open part is higher than a rate of the added liquid sample permeating the downstream region of the chromatography specimen 1, the liquid sample is dried without flowing back. Further, the liquid sample is applied to the sample application part 2 and developed toward the downstream region of the chromatography specimen 1 so as to make proportion of moisture uniform in the upstream and downstream region of the chromatography specimen 1, and a reaction is performed accurately in the reactive layer 4.

As described above, according to the chromatography measuring device in the first embodiment, a region at least from the marker reagent holding part 3 to the specific protein immobilization part 5 on the surface of the chromatography specimen 1, except for both of its ends on chromatographic upstream and downstream, is adherently covered with.the liquid-impermeable sheet material 6, thereby enabling chromatographic development without absorbing the developed sample liquid employing a member having high water absorption in the downstream region of the chromatography specimen. Further, the chromatography specimen 1 comprises the sample application part 2, the marker reagent holding part 3, the specific protein immobilization part 5, and the reactive layer 4, and the chromatography measuring device further has the support body 7 for supporting these constituent members, thereby realizing a low-cost chromatography measuring device having high sensitivity and high performance, whose constituent members are to be reduced and whose specimen manufacturing process is simplified.

While, in the chromatography measuring device according to the present invention, the description has been given of the case where there is one region for the specific protein immobilization part 5 on the reactive layer 4, the region is not necessarily required to be one and the arbitrary number of specific protein immobilization regions may exist on the region of the reactive layer 4. Further, the description has been given employing the specific binding protein as the reactive component employed for the chromatography measurement, the reactive component is not restricted to this and substance, such as an enzyme, which causes some changes before and after the reaction may be employed as the reactive component employed for the chromatography measurement.

Further, while plastic or the like has been taken as an example of the composition of the support body 7, an arbitrary liquid-impermeable material such as vinyl tape and PET (Polyethylene Terephthalate) is preferred. Further, a nonwoven fabric and nitrocellulose are taken just as examples of the compositions of the sample application part 2, marker reagent holding part 3, and reactive layer 4 and may be composed of an arbitrary porous support.

Further, in the present invention the chromatography specimen 1 constituting the chromatography measuring device is constituted by laminating or connecting arbitrary plural porous materials such as nitrocellulose or a glass fiber filter. The chromatography specimen composed of such materials can analyze, detect, and quantitatively measure specific substance employing an arbitrary measurement principle such as an antigen-antibody reaction. Further, it is possible to measure a liquid sample such as water, an aqueous solution, urine, blood, body fluid, and a solution in which solid, fine particles, or gas are dissolved, and its applications include urinalysis, a pregnancy test, a water examination, an examination of the feces, soil analysis, food analysis, and the like.

### (Embodiment 2)

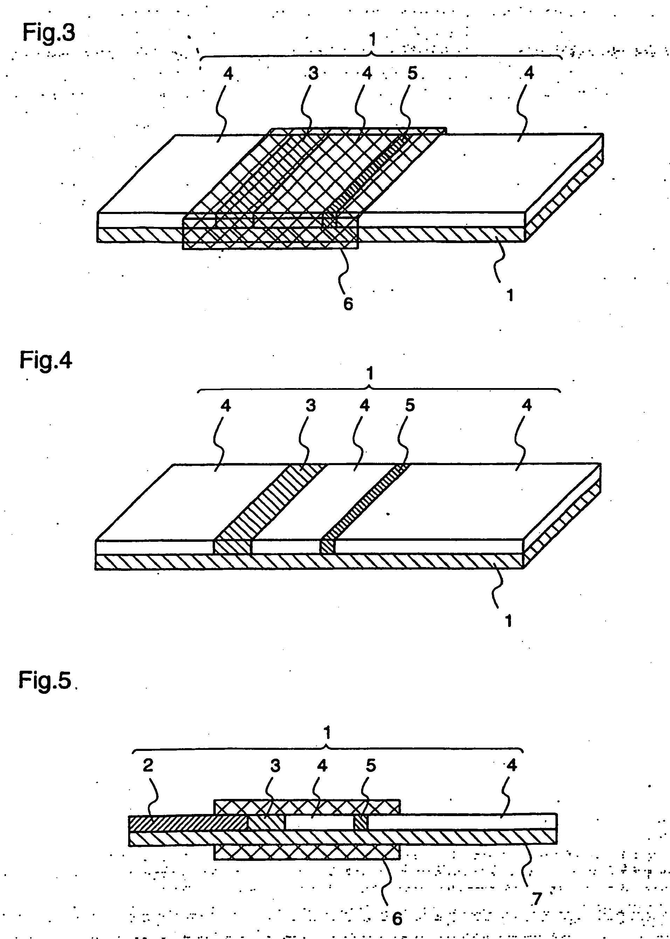

Hereinafter, a chromatography measuring device according to a second embodiment of the present invention will be described with reference to figures 3 and 4.

Figure 3 is a diagram illustrating the chromatography measuring device according to the second embodiment of the present invention and figure 4 illustrates a chromatography specimen without a liquid-impermeable sheet material of the chromatography, measuring device.

Figure 3 illustrates the chromatography measuring device for qualitatively or quantitatively measuring substance to be tested, which is applied to a chromatography specimen 1, and the chromatography specimen 1 is adherently covered with a liquid-impermeable sheet material 6 except for both of its end regions on chromatographic upstream and downstream.

Figure 4 differs from the chromatography specimen 1 according to the first embodiment described with reference to figure 2 only in that a sample application part 2 is not provided and a liquid sample is directly added or applied onto a reactive layer 4, so that a basic constituent member of the chromatography specimen is composed of a single layer of the reactive layer 4 only. Therefore, the same constituents as those in the chromatography measuring device according to the aforementioned first embodiment will be denoted by the same reference numerals, and thus their descriptions will be omitted.

Next, a chromatography measurement employing the chromatography measuring device (See figure 3) according to the second embodiment of the present invention will be described.

In the chromatography measuring device shown in figure 3, a liquid sample is applied to the reactive layer 4, and then it reaches the region of the marker reagent holding part 3. Then, a marker reagent held in the region of the marker reagent holding part 3 is dissolved due to the permeation of the liquid sample and permeates the region of the specific protein immobilization part 5 with the liquid sample. When the liquid sample includes an analyte therein, a specific protein immobilized in the specific protein immobilization part 5 performs a binding reaction with a complex of the analyte and the marker reagent, and a color reaction is seen in the region of the specific protein immobilization part 5. On the other hand, when the liquid sample does not include an analyte therein, no binding reaction is caused and no color reaction is seen. The liquid sample finally permeates a bottom region of the chromatography specimen 1, and the reaction is ended.

At this time, a measurement region at least from the marker reagent holding part 3 located upstream to the specific protein immobilization part 5 located downstream on the surface of the chromatography specimen 1 is adherently covered with the liquid-impermeable sheet material 6 made of plastic tape or the like, thereby preventing evaporation of moisture in the measurement region, making the amount of the liquid sample permeating the measurement area uniform, and making concentrations of the liquid sample and marker reagent flowing in the measurement region for a definite period of time constant, resulting in an accurate chromatography measurement.

Further, the downstream region of the chromatography specimen 1 is not covered with the liquid-impermeable sheet material 6, whereby moisture is gradually evaporated as the liquid sample permeates a bottom open part as a region where the bottom of the chromatography specimen 1 is opened. Since an evaporation rate of the liquid sample in the bottom open part is higher than a rate of the added liquid sample permeating the downstream region of the chromatography specimen. 1, the liquid sample is dried without flowing back.

Further, the liquid sample is directly added or applied onto the reactive layer 4, so that the sample application part 2 need not be provided and the basic members of the chromatography specimen 1 can be composed of a single-layer porous material of the reactive layer 4 only, thereby reducing the number of constituent members for constituting the chromatography measuring device. Further, when the single-layer porous material is composed of nitrocellulose, only a minute amount of the liquid sample application is sufficient, resulting in a chromatography measuring device which uses only minute amount of analyte.

As described above, according to the second embodiment, the sample application part 2 is not provided, and the arbitrary surface of the chromatography specimen 1 composed of a single-layer porous material, except for both of its ends on chromatographic upstream and downstream, is adherently covered with the liquid-impermeable sheet material 6 composed of plastic tape or the like, so that there is no need to provide a water-absorbing part for absorbing a sample at a downstream region of the chromatography specimen 1, thereby, realizing a low-cost chromatography measuring device having high sensitivity and high performance, whose constituent members are to be reduced and whose specimen manufacturing process-is simplified.

While the description has been given of the chromatography measuring device in which the sample application part 2 is not provided and a liquid sample is directly added or applied onto the reactive layer 4, the reactive layer 4 is composed of a porous material, and thus a measurement can be performed sufficiently only with a minute amount of the sample liquid addition.

### (Embodiment 3)

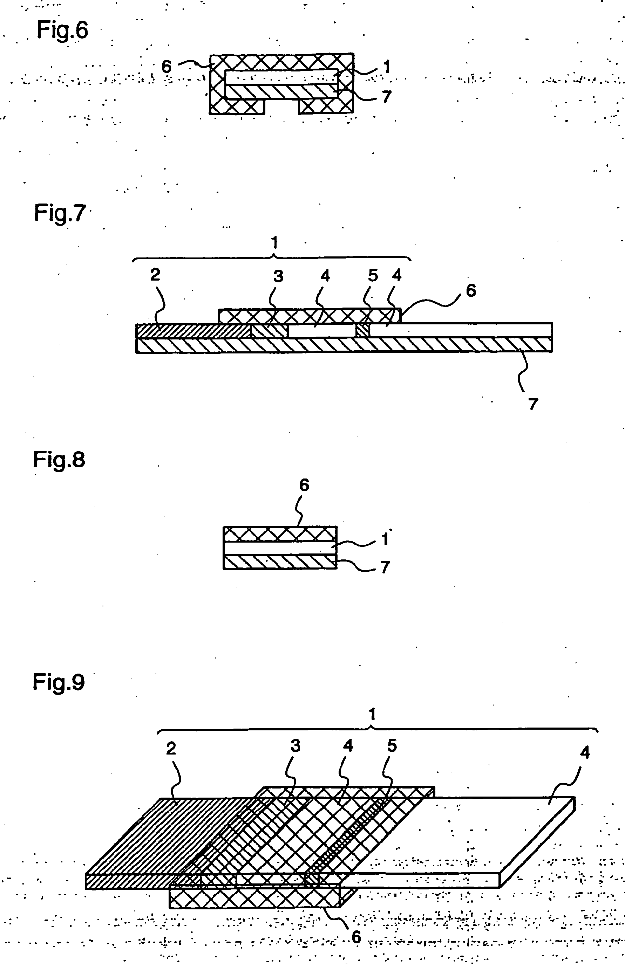

Hereinafter, a covering state in which the chromatography specimen 1 is covered with the liquid-impermeable sheet material 6 in the chromatography measuring device according to the first embodiment will be described with reference to figures 5 and 6.

Figures 5 and 6 are diagrams for explaining the covering state of the liquid-impermeable sheet material 6 of the chromatography measuring device in figure 1. Figure 5 is a sectional view of the chromatography measuring device shown in figure 1, which is cut parallel to, the direction of sample liquid permeation (hereinafter, referred to as the chromatographic direction), and figure 6 is a sectional view of the chromatography measuring device shown in figure 1, which is cut perpendicular to the chromatographic direction.

Further, the same constituents as those in the chromatography measuring device according to the aforementioned first embodiment will be denoted by the same reference numerals, and thus their descriptions will be omitted.

At this time, the top surface and side faces of a measurement region at least from the marker reagent holding part 3 located upstream to the specific protein immobilization part 5 located downstream on the surface of the chromatography specimen 1 are adherently covered with the liquid-impermeable sheet material 6 made of plastic tape or the like, thereby preventing evaporation of moisture in the measurement region, making the amount of the liquid sample permeating the measurement area uniform, and further making concentrations of the liquid sample and marker reagent flowing in the measurement region for a definite period of time constant, resulting in an accurate chromatography measurement.

Further, the downstream region of the chromatography specimen 1 is not covered with the liquid-impermeable sheet material 6, whereby moisture is gradually evaporated as the liquid sample permeates a bottom open part as a region where the bottom of the chromatography specimen 1 is opened. Since an evaporation rate of the liquid sample in the bottom open part is higher than a rate of the added liquid sample permeating the downstream region of the chromatography specimen 1, the liquid sample is dried without flowing back. Further, the liquid sample is applied to the sample application part 2 and developed toward the downstream region of the chromatography specimen 1 so as to make proportion of moisture uniform in the upstream and downstream region of the chromatography specimen 1, and a reaction is performed accurately in the reactive layer 4.

As described above, according to the chromatography measuring device in the third embodiment, the top surface and the side faces of the measurement region at least from the marker reagent holding part 3 located upstream to the specific protein immobilization part 5 located downstream on the surface of the chromatography measuring device are adherently covered with the liquid-impermeable sheet material 6 made of plastic tape or the like, whereby evaporation of moisture in the measurement region can be prevented, so that chromatographic development occurs uniformly, resulting in an accurate chromatography measurement, and there is no need to provide a water-absorbing part for absorbing a liquid sample at a downstream region of the chromatography specimen 1, resulting in a low-cost chromatography measuring device having high sensitivity and high performance, whose constituent members are to be reduced and whose specimen manufacturing process is simplified.

While in the third embodiment the description has been given of the covering state of the liquid-impermeable sheet material 6 of the chromatography measuring device according to the first embodiment, the top surface and side surfaces of the chromatography measuring device according to the second embodiment may be also covered with the liquid-impermeable sheet material 6, thereby to achieve the same effects as those in the third embodiment.

### (Embodiment 4)

Hereinafter, a covering state in which the chromatography specimen 1 is covered with the liquid-impermeable sheet material 6 in the chromatography measuring device according to the aforementioned first and second embodiments will be described with reference to figures 7 and 8.

Figures 7 and 8 are diagrams for explaining a covering state of the liquid-impermeable sheet material 6 of the chromatography measuring device shown in figure 1. Figure 7 is a sectional view of the chromatography measuring device shown in figure 1, which is cut parallel to the chromatographic direction, and figure 8 is a sectional view of the chromatography measuring device shown in figure 1, which is cut perpendicular to the chromatographic direction. Further, the same constituents as those in the chromatography measuring device according to the aforementioned first embodiment will be denoted by the same reference numerals, and thus their descriptions will be omitted.

At this time, the top surface of a measurement region at least from the marker reagent holding part 3 located upstream to the specific protein immobilization part 5 located downstream on the surface of the chromatography specimen 1 is adherently covered with the liquid-impermeable sheet material 6 made of plastic tape or the like, thereby preventing evaporation of moisture in the measurement region, making the amount of the liquid sample permeating the measurement area uniform, and making concentrations of the liquid sample and marker reagent flowing in the measurement region for a definite period of time constant, resulting in an accurate chromatography measurement.

Further, the downstream region of the chromatography specimen 1 is not covered with the liquid-impermeable sheet material 6, whereby moisture is gradually evaporated as the liquid sample permeates a bottom open part as a region where the bottom of the chromatography specimen 1 is opened. Since an evaporation rate of the liquid sample in the bottom open part is higher than a rate of the added liquid sample permeating the downstream region of the chromatography specimen 1, the liquid sample is dried without flowing back. Further, the liquid sample is applied to the sample application part 2 and the liquid sample is developed toward the downstream region of the chromatography specimen 1 so as to make proportion of moisture uniform in the upstream and downstream region of the chromatography specimen 1, and a reaction is performed accurately in the reactive layer 4.

Since the liquid-impermeable sheet material 6 adherently covers only the top surface of the chromatography measuring device as shown in figure 8, there is no need to wrap the liquid-impermeable sheet material 6 around the chromatography specimen 1 to its back side at manufacture of the chromatography measuring device, and further there is no need to worry about exfoliation of the members at cutting when the manufactured chromatography sheet is cut into the chromatography specimen size, since the surface of the chromatography sheet is covered adherently with the liquid-impermeable sheet material 6.

As described above, according to the chromatography measuring device in the fourth embodiment, the top surface of the measurement region at least from the marker reagent holding part 3 located upstream to the specific protein immobilization part 5 located downstream on the surface of the chromatography measuring device is adherently covered with the liquid-impermeable sheet material 6 made of plastic tape or the like, whereby evaporation of moisture in the measurement region can be prevented, so that chromatographic development occurs uniformly, resulting in an accurate chromatography measurement, and there is no need to provide a water-absorbing part for absorbing a liquid sample at a downstream region of the chromatography specimen 1, resulting in a low-cost chromatography measuring device having high sensitivity and high performance, whose constituent members are to be reduced and whose specimen manufacturing process is simplified.

While in the fourth embodiment the description has been given of the covering state of the liquid-impermeable sheet material 6 of the chromatography measuring device according to the first embodiment, the surface of the chromatography measuring device according to the second embodiment may be also covered with the liquid-impermeable sheet material 6, thereby to achieve the same effects as those in the fourth embodiment.

### (Embodiment 5)

Hereinafter, a chromatography measuring device according to a fifth embodiment of the present invention will be described with reference to figures 9, 10, and 11.

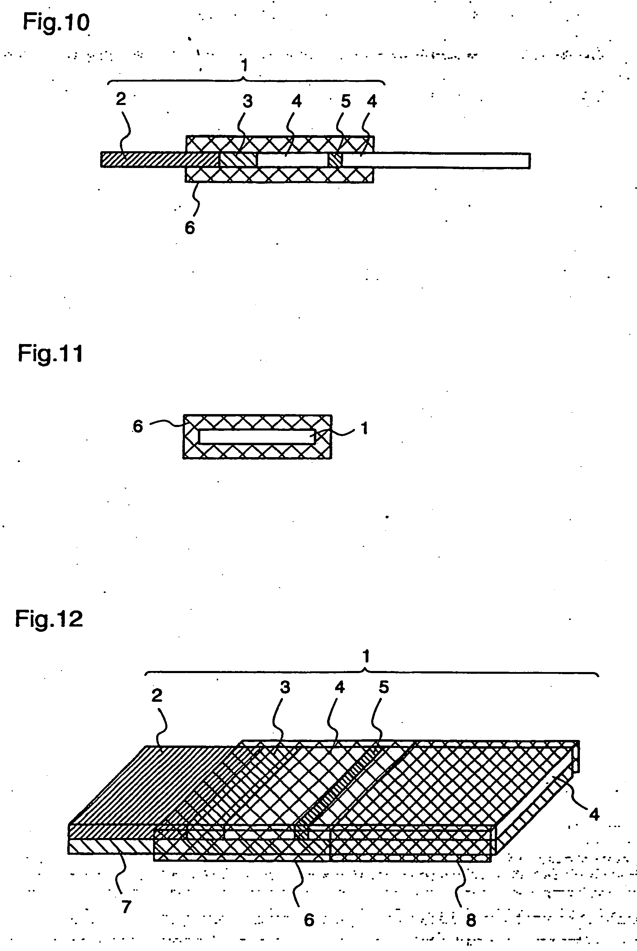

Figure 9 is a diagram illustrating the chromatography measuring device according to the fifth embodiment of the present invention. Figures 10 and 11 are diagrams for explaining a covering state of the liquid-impermeable sheet material 6 of the chromatography measuring device shown in figure 9. Figure 10 is a sectional view of the chromatography measuring device shown in figure 9, which is cut parallel to the chromatographic direction, and figure 11 is a sectional view of the chromatography measuring device shown in'figure 9, which is cut perpendicular to the chromatographic direction.

In the chromatography measuring device according to the fifth embodiment, the top surface, side faces, and bottom surface of the chromatography specimen 1 are adherently covered with the liquid-impermeable sheet material 6 as shown in figure 9, there is no need to provide a support body 7. That is, constituent members of the chromatography measuring device according to the fifth embodiment differs from those of the chromatography measuring device according to the first embodiment described with reference to figure 1 only in that the support body 7 is not provided. Therefore, the same constituents as those in the chromatography measuring device according to the aforementioned first embodiment will be denoted by the same reference numerals, and thus their descriptions will be omitted.

Next, a chromatography measurement employing the chromatography measuring device (See figure 9) according to the fifth embodiment of the present invention will be described.

In the chromatography measuring device shown in figure 9, a liquid sample is applied to the sample application part 2, and then it reaches the region of the marker reagent holding part 3. Then, a marker reagent held in the region of the marker reagent holding part 3 is dissolved due to the permeation of the liquid sample and permeates the reactive layer 4 with the liquid sample. On the region of the reactive layer 4, there is the specific protein immobilization part 5, and when the liquid sample includes an analyte therein, a specific protein immobilized in the specific protein immobilization part 5 performs a binding reaction with a complex of the analyte and the marker reagent, and a color reaction is seen in the region of the specific protein immobilization part 5. On the other hand, when the liquid sample does not include an analyte therein, no binding reaction is caused and no color reaction is seen. The liquid sample finally permeates a bottom region of the chromatography specimen 1, and the reaction is ended.

At this time, the top surface, side faces, and bottom surface of a measurement region at least from the marker reagent holding part 3 located upstream to the specific protein immobilization part 5 located downstream on the surface of the chromatography specimen 1 is adherently covered with the liquid-impermeable sheet material 6 made of plastic tape or the like, thereby preventing evaporation of moisture in the measurement region, making the amount of the liquid sample permeating the measurement area uniform, and making concentrations of the liquid sample and marker reagent flowing in the measurement region for a definite period of time constant, resulting in an accurate chromatography measurement.

Further, the downstream region of the chromatography specimen 1 is not covered with the liquid-impermeable sheet material 6, whereby moisture is gradually evaporated as the liquid sample permeates a bottom open part as a region where the bottom of the chromatography specimen 1 is opened. Since an evaporation rate of the liquid sample in the bottom open part is higher than a rate of the added liquid sample permeating the downstream region of the chromatography specimen 1, the liquid sample is dried without flowing back. Further, the liquid sample is applied to the sample application part 2 and the liquid sample is developed toward the downstream region of the chromatography specimen 1 so as to make proportion of moisture uniform in the upstream and downstream region of the chromatography specimen 1, and a reaction is performed accurately in the reactive layer 4.

Further, the liquid-impermeable sheet material 6 also serves as the support body 7, thereby reducing the number of the constituent members for constituting the chromatography measuring device.

As described above, according to the chromatography measuring device in the fifth embodiment, the arbitrary surface of the chromatography specimen 1, except for both of its ends on chromatographic upstream and downstream, is adherently covered with the liquid-impermeable sheet material 6 composed of plastic tape-or the like, whereby evaporation of moisture in the measurement region can be prevented, so that chromatographic development occurs uniformly, resulting in an accurate chromatography measurement, and there is no need to provide a water-absorbing part for absorbing a liquid sample at a downstream region of the chromatography specimen 1. Further, the top surface, side faces, and bottom surface of the chromatography specimen 1 is covered adherently with the liquid-impermeable sheet material 6, whereby there is no need to provide the support body 7, resulting in a low-cost chromatography measuring device having high sensitivity and high performance, whose constituent members are to be reduced and specimen manufacturing process is simplified.

While.in the fifth embodiment the description has been given employing the constitution of the chromatography specimen 1 of the chromatography measuring device according to the first embodiment, the chromatography specimen 1 described in the aforementioned second embodiment, which has its basic constituent member composed of a single layer of the reactive layer 4 only, can also achieve the same effects as those in the fifth embodiment.

### (Embodiment 6)

Hereinafter, a chromatography measuring device according to a sixth embodiment of the present invention will be described with reference to figure 12.

Figure 12 is a diagram illustrating the chromatography measuring device according to the sixth embodiment of the present invention.

Figure 12 illustrates the chromatography measuring device for qualitatively or quantitatively measuring substance to be tested, which is applied to a chromatography specimen 1, and the chromatography specimen 1 is adherently covered with a liquid-impermeable sheet material 6 except for both of its end regions on chromatographic upstream and downstream.

As shown in figure 12, the chromatography measuring device according to the sixth embodiment of the present invention differs from the chromatography measuring device according to the first embodiment described with reference to figure 1 only in that the chromatographic downstream region which is not covered with the liquid-impermeable sheet material 6 is covered with a perforated material 8. Therefore, the same constituents as those in the chromatography measuring device according to the aforementioned first embodiment will be denoted by the same reference numerals, and thus their descriptions will be omitted.

The perforated material 8 may be any mesh-form materials having pores, preferably materials having a low water absorption.

Next, a chromatography measurement employing the chromatography measuring device according to the sixth embodiment of the present invention will be described.

In the chromatography measuring device shown in figure 12, a liquid sample is applied to the sample application part 2, and then it reaches the region of the marker reagent holding part 3. Then, a marker reagent held in the region of the marker reagent holding part 3 is dissolved due to the permeation of the liquid sample and permeates the region of the reactive layer 4 with the liquid sample. On the region of the reactive layer 4, there is the specific protein immobilization part 5, and when the liquid sample includes an analyte therein, a specific protein immobilized in the specific protein immobilization part 5 performs a binding reaction with a complex of the analyte and the marker reagent, and a color reaction is seen in the region of the specific protein immobilization part 5. On the other hand, when the liquid sample does not include an analyte therein, no binding reaction is caused and no color reaction is seen. The liquid sample finally permeates a bottom region of the chromatography specimen, and the reaction is ended.

At this time, a measurement region at least from the marker reagent holding part 3 located upstream to the specific protein immobilization part 5 located downstream on the surface of the chromatography specimen 1 is adherently covered with the liquid-impermeable sheet material 6 made of plastic tape or the like, thereby preventing evaporation of moisture in the measurement region, making the amount of the liquid sample permeating-the measurement area uniform, and making concentrations of the liquid sample and marker reagent flowing in the measurement region for a definite period of time constant, resulting in an accurate chromatography measurement.

Further, the downstream region of the chromatography specimen 1 is not covered with the liquid-impermeable sheet material 6 but with the perforated material 8, whereby moisture is gradually evaporated as the liquid sample permeates the bottom region of the chromatography specimen 1. Since an evaporation rate of the liquid sample in the bottom region is higher than a rate of the added liquid sample permeating the downstream region of the chromatography specimen 1, the liquid sample is dried without flowing back. The perforated material may also be an arbitrary porous thin-film material such as nonwoven fabric, and as the porous thin-film material, one which is excellent in gas permeability because of porousness and is 1mm thick or less is preferably used and further its own water absorption is preferably low. Further, the perforated material may also be retiform tissue. The retiform tissue described here is formed reticulately by a fiber or resin molding processing and presence or absence of its own capillary activity or water absorption does not mater. The reticulated form at this time may be of any forms as long as it is a polygon and its reticulum is preferably aligned regularly, while its size does not matter. Further, this retiform tissue is preferably of a single layer.

Further, the liquid sample is applied to the sample application part 2 and the liquid sample is developed toward the downstream region of the chromatography specimen 1 so as to make proportion of moisture uniform in the upstream and downstream region of the chromatography specimen 1, and a reaction is performed accurately in the reactive layer 4.

Further, since the perforated material 8 covers the chromatographic downstream region as shown in figure 12, the sample application part 2 can be recognized visually and the reactive layer 4 at the downstream region can not be touched directly with one's hand. Accordingly, there is no possibility that the reactive layer 4 is polluted by substance attached to one's hand, resulting in no need of worrying about deterioration in water absorption in the reactive layer 4 which is caused by pollution or worrying about the liquid sample being attached to one's hand, thereby performing a measurement safely and sanitarily.

As described above, according to the chromatography measuring device in the sixth embodiment, the measurement region at least from the marker reagent holding part 3 located upstream to the specific protein immobilization part 5 located downstream on the surface of the chromatography measuring device is adherently covered with the liquid-impermeable sheet material 6 made of plastic tape or the like, whereby evaporation of moisture in the measurement region can be prevented, so that chromatographic development occurs uniformly, resulting in an accurate chromatography measurement. Further, the downstream region of the chromatography specimen 1, is covered with the perforated material 8, so that moisture evaporation is prompted and the sample application part 2 can be easily recognized, thereby preventing deterioration in water absorption in the reactive layer due to pollutant. Therefore, it is possible to realize a low-cost chromatography measuring device having high sensitivity and high performance, which can be used safely and sanitarily with no liquid sample attached to one' s hand.

While, in the chromatography measuring device according to the present invention, the description has been given of the case where the perforated material 8 covers the downstream region, as long as the perforated material 8 exists in the downstream region, any patterns are available, such as the one in which the top surface and side faces of the downstream region are covered with the perforated material 8, the one in which only the surface is covered with the perforated material 8, or the one in which the top surface, side faces, and bottom surface are covered with the perforated material 8, as described in the third to fifth embodiments.

While in the sixth embodiment the description has been given employing the constitution of the chromatography specimen 1 of the chromatography measuring device according to the first embodiment, the chromatography, specimen 1 described in the second embodiment, which has its basic constituent member composed of a single layer of the reactive layer 4 only, can also achieve the same effects as those in the sixth embodiment.

### (Embodiment 7)

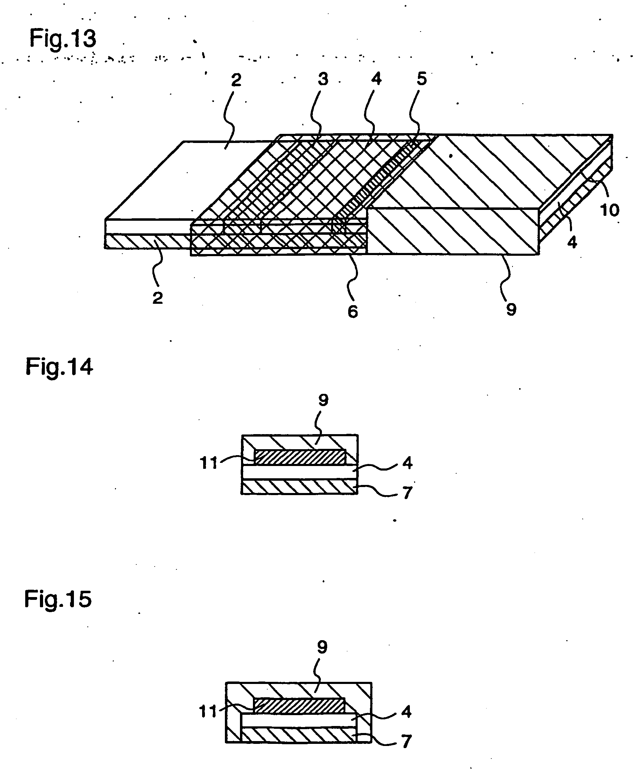

Hereinafter, a chromatography measuring device according to a seventh embodiment of the present invention will be described with reference to figures 13, 14, 15, 16, 17, and 18.

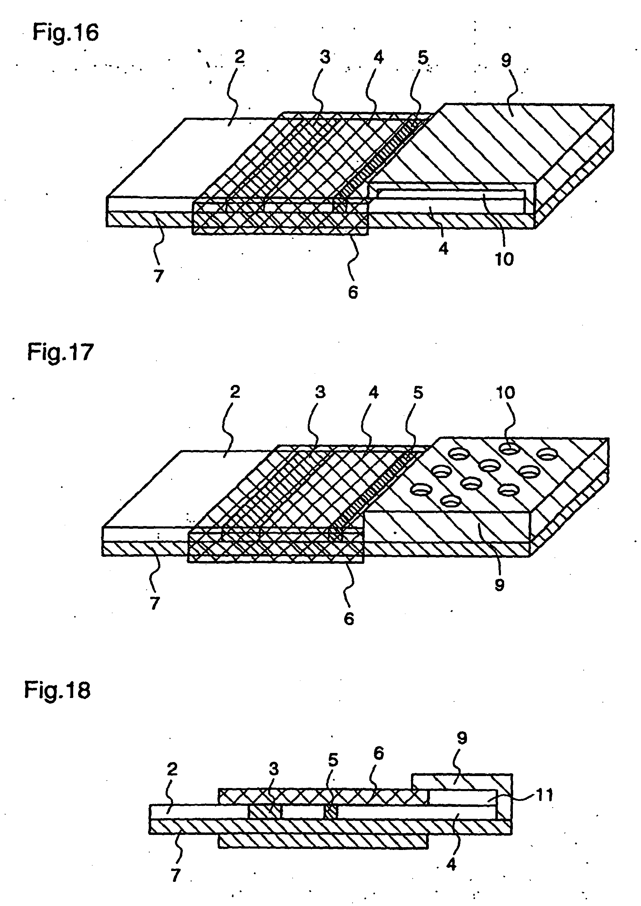

Figures 13, 16, and 17 are diagrams illustrating the chromatography measuring device according to the seventh embodiment of the present invention, and figures 14 and 15 are diagrams of the cross sections of the chromatography measuring device shown in figure 13, which is cut perpendicular to the chromatographic direction, which are seen from the chromatographic downstream direction. Further, figure 18 is a cross sectional view of the chromatography measuring device shown in figure 16, which is cut parallel to the chromatographic direction.

Figures 13, 16, and 17 illustrate the chromatography measuring device for qualitatively or quantitatively measuring substance to be tested which is applied to a chromatography specimen 1, and the chromatography specimen 1 is adherently covered with a liquid-impermeable sheet material 6 except for both of its end regions on chromatographic upstream and downstream.

As shown in figures 13, 16, and 17, the chromatography measuring device according to the seventh embodiment of the present invention differs from the chromatography measuring device according to the first embodiment described with reference to figure 1 only in that the chromatographic downstream region which is not covered with the liquid-impermeable sheet material 6 is covered with a space forming material 9. Therefore, the same constituents as those in the chromatography measuring device according to the first embodiment will be denoted by the same reference numerals, and thus their descriptions will be omitted.

The space forming material 9 is an arbitrary liquid-impermeable material, preferably a material with enough strength to form and maintain space on the reactive layer 4, while it may be any of transparent, semitransparent or opaque ones. At this time, the space forming material 9 may be formed to be laminated on an end of the liquid-impermeable sheet material 6 as shown in figure 18, or to be connected with the liquid-impermeable sheet material 6, while not shown.

A space forming part 11 is space formed by the space forming material 9, which provides arbitrary space intervals between itself and the reactive layer 4 and preferably air can inflow therein. '

A gap part 10 can be provided at arbitrary region of the space forming material 9 in the arbitrary numbers, such as at the chromatographic downstream end as shown in figure 13, on a chromatographic parallel side as shown in figure 16, and on the surface of the space, forming part 11 as shown in figure 17. Further, the gap part described here may be provided in one or the arbitrary-number which is mo\re than one.

Next, a chromatography measurement employing the chromatography measuring devices according to the seventh embodiment of the present invention will be described.

In the chromatography measuring device shown in figures 13, 16, and 17, a liquid sample is applied to the sample application part 2, and then it reaches the region of the marker reagent holding part 3. Then, a marker reagent held in the region of the marker reagent holding part 3 is dissolved due to the permeation of the liquid sample and permeates the region of the reactive layer 4 with the liquid sample. On the region of the reactive layer 4, there is the specific protein immobilization part 5, and when the liquid sample includes an analyte therein, a specific protein immobilized in the specific protein immobilization part 5 performs a binding reaction with a complex of the analyte and the marker reagent, and a color reaction is seen in the region of the specific protein immobilization part 5. On the other hand, when the liquid sample does not include an analyte therein, no binding reaction is caused and no color reaction is seen. The liquid sample finally permeates a bottom region of the chromatography specimen, and the reaction is ended.

At this time, a measurement region at least from the marker reagent, holding part 3 located upstream to the specific protein immobilization part 5 located downstream on the surface of the chromatography specimen 1 is adherently covered with the liquid-impermeable sheet material 6 made of plastic tape or the like, thereby preventing evaporation of moisture in the measurement region, making the amount of the liquid sample permeating the measurement area uniform, and.making concentrations of the liquid sample and marker reagent flowing in the measurement region for a definite period of time constant, resulting in an accurate chromatography measurement.

Further, the downstream region of the chromatography specimen 1 is not covered with the liquid-impermeable sheet material 6 but with the space forming material 9, whereby moisture is gradually evaporated as.the liquid sample permeates the bottom region of the chromatography specimen 1. The space forming material 9 has the gap part 10, thereby an evaporation rate of the liquid sample in the bottom region is higher than a rate of the added liquid sample permeating the downstream region of the chromatography specimen 1, whereby the liquid sample is dried without flowing back. Further, the liquid sample is applied to the sample application part.2 and the liquid sample is developed toward the downstream region of the chromatography specimen. 1 so as to make proportion of moisture uniform in the upstream and downstream region of the chromatography specimen 1, and a reaction is performed accurately in the reactive layer 4.

Further, since the space forming material 9 covers the chromatographic downstream region as shown in figures 13, 16, and 17, the sample application part 2 can be recognized visually and the reactive layer 4 at the downstream region can not be touched directly with one's hand. Accordingly, there is no possibility that the reactive layer 4is polluted by substance attached to one's hand, resulting in no need of worrying about deterioration in water absorption in the reactive layer 4 which is caused by pollution or worrying about the liquid sample being attached to one's hand, thereby performing a measurement safely and sanitarily.

As described above, according to the chromatography measuring device in the seventh embodiment, the measurement region at least from the marker reagent holding part 3 located upstream to the specific protein immobilization part 5 located downstream on the surface of the chromatography measuring device is adherently covered with the liquid-impermeable sheet material 6 made of plastic tape or the like, whereby evaporation of moisture in the measurement region can be prevented, so that chromatographic development occurs uniformly, resulting in an accurate chromatography measurement. Further, the downstream region of the chromatography specimen 1 is covered with the space forming material 9, so that moisture evaporation is prompted and the sample application part 2 can be easily recognized, thereby preventing deterioration in water absorption in the reactive layer due to pollutant. Therefore, it is possible to realize a low-cost chromatography measuring device having high sensitivity and high performance, which can be used safely and sanitarily with no liquid sample attached to one' s hand.

While, in the chromatography measuring device according to the present invention, the description has been given of the case where the space forming material 9 covers the downstream region, the space forming material 9 may be specifically formed on the surface of the reactive layer as shown in figure 14. Further, as long as the space forming material 9 exists in the downstream region, any patterns are available, such as the one in which the space forming material 9 is formed on the top surface and side faces of the chromatography measuring device as shown in figure 15, or the one in which the space forming material 9 is formed on the top surface, side faces, and bottom surface, while not shown.

While in the seventh embodiment the description has been given employing the constitution of the chromatography specimen 1 of the chromatography measuring device according to the first embodiment, the chromatography specimen 1 described in the second embodiment, which has its basic constituent member composed of a single layer of the reactive layer 4 only, can also achieve the same effects as those in the seventh embodiment.

Further, in the chromatography measuring device according to the present invention, its specimen may be a dry analysis element. The dry analysis element described here indicates a specimen in which its constituent members such as the sample application part, the reactive layer, and the water-absorbing part are in a dry state, and reagents such as a sample reagent and a specific protein supported or immobilized on the members are also constituted in a dry state. When the specimen is in a dry state in this way, denaturation of a protein or the like can be minimized and long-term storage is possible.

Further, the chromatography measuring device according to the present invention may have a one-step specimen. One-step described here is an operation in which a pretreatment of a sample solution is not required, and, the sample solution is only applied to a specimen, and it is not required to develop the sample solution employing a development solution after the application or to perform a washing operation at a measurement operation, thereby simplifying a measurement.

### Example

A method for implementing the present invention will be described in more detail through a following example. The present invention is not restricted to the following example. (Qualitative analysis of hCG in urine)

An immunochromatography-measuring device which includes an anti-hCG-ß antibody, immobilization line and a broad band of a complex of an anti-hCG-α antibody and gold colloid in a nitrocellulose film is manufactured. This chromatography measuring device is shown in figure 1. In this figure, the chromatography measuring device includes the specific protein immobilization part 5 in which the antibody is immobilized, the marker reagent holding region 3 positioned prior to the specific protein immobilization part 5, which is an area including the complex of the anti-hCG-a antibody and the gold colloid, and the sample application part 2.

These chromatography measuring devices are manufactured as follows.

### a) Preparation of chromatography measuring device

An anti-hCG-β antibody solution which was diluted with a phosphoric acid buffer solution to control the concentration was prepared. This antibody solution was applied on the nitrocellulose film by employing a solution discharge device, thereby, obtaining an antibody immobilization line for detection on the film. After this film is dried, this film was immersed in a Tris-HCl buffer solution including 1% skim milk and shaken gently for 30 minutes. After 30 minutes have passed, the film was moved into a Tris-HCl buffer solution tank, shaken gently; for 10 minutes, and thereafter shaken gently in another Tris-HCl buffer solution tank for another 10 minutes, to wash the film. After washed twice, the film was taken out from the solution tank and dried at room temperatures.

The gold colloid was prepared by adding 1% citric acid solution to 100°C-solution of 0.01% gold chloride acid while being refluxed. After the reflux was continued for 30 minutes, it was cooled being left at room temperature. The anti-hCG-α antibody was added to gold colloid solution which was prepared to pH9 by using 0.2M potassium carbonate solution, to be stirred for several minutes, and then 10% BSA (bovine serum albumin) solution of pH9 was added thereto by such an amount that 1% solution was finally obtained and stirred. Thereby, an antibody-gold colloid complex (marker antibody) was prepared. The marker antibody solution was centrifuged at 4°C and 20000G for 50 minutes, whereby the marker antibody was isolated, and the isolated marker antibody was suspended in a washing buffer solution (1% BSA · phosphoric acid buffer solution) and thereafter centrifuged again to wash and isolate the marker antibody. After this marker antibody is suspended in the washing buffer solution and filtrated employing a 0.8pm filter, the marker antibody was prepared to be one-tenth as much as the initial gold colloid solution and stored at 4°C.

The marker antibody solution was set in the solution discharge device and applied to a position on an anti-hCG-β antibody immobilization dry film, apart from an antibody immobilization position, and thereafter the film was dried. Thereby, the chromatography specimen 1 having the marker antibody holding region 3 was obtained on the antibody immobilization film.

Then, this chromatography specimen 1 is attached to the support body 7 and thereafter transparent tape (Nitto Denko Corporation made) covers the chromatography specimen 1 from a position which is 1.5cm apart from the upstream end to a position which is 1.0cm apart from the downstream end so as to adhere to the surface of the reactive layer, thereby obtaining the chromatography measuring device.

### b) Preparation of sample

Human male urine including no hCG was prepared. The hCG solutions of certain concentrations were added to the urine, thereby preparing the hCG solutions of various known concentrations..

### c) Measurement of the degree of coloration on chromatography measuring device

About 40µl of urine including hCG was applied to the sample application part on the chromatography measuring device and developed toward the downstream of the chromatography specimen 1, to be subjected to an antigen-antibody reaction, whereby a color reaction in the antibody immobilization part was caused. The coloration state when 5 minutes have passed since the sample application to this measuring device was visually judged.

As a result, the sample is steadily developed chromatographically without flowing back, and the color reaction caused by existence of hCG can be confirmed.

While the gold colloid is employed as the marker reagent in this example, any markers are available as long as the marker reagents can color.

Further, the chromatography measuring device according to this example employs the specimen constituted by laminating or connecting arbitrary porous materials such as nitrocellulose as described above. The specimen composed of such materials can analyze, detect, and qualitatively or quantitatively measure specific substance employing an arbitrary measurement principle such as an antigen-antibody reaction.

### APPLICABILITY IN INDUSTRY

As described above, according to a chromatography measuring device of the present invention, chromatographic development occurs uniformly and a chromatography measurement is performed accurately, as well as reduce its constituent members, so that a liquid sample such as an aqueous solution, urine, and blood can be measured with high sensitivity and efficiency, and therefore the chromatography measurement device is suitable for urinalysis, a pregnancy test, a water examination, an examination of the feces, soil analysis, food analysis, and the like.

## Claims

1. A chromatography measuring device which has a chromatography specimen as a specimen for performing a chromatography measurement and qualitatively or quantitatively measures substance to be tested, which is applied to the chromatography specimen, wherein
the chromatography specimen is open over at least the top and side surfaces of both end regions on chromatographic upstream and downstream, and is adherently covered with a liquid-impermeable sheet material at all parts of the top surface except for both of its end regions on chromatographic upstream and downstream.

2. The chromatography measuring device as defined in Claim 1, wherein
the chromatography specimen is further adherently covered with a liquid-impermeable sheet material at all parts of the side surfaces except for both of its end regions on chromatographic upstream and downstream.

3. The chromatography measuring device as defined in Claim 1, wherein . '
the chromatography specimen is further adherently covered with a liquid-impermeable sheet material at all parts of the side surfaces and bottom surface except for both of its end regions on chromatographic upstream and downstream.

4. The chromatography measuring device as defined in any of Claims 1 to 3, wherein
a measurement region at least from a marker reagent holding part in which a marker reagent is held, located upstream, to a specific protein immobilization part in which a specific protein is immobilized, located downstream, in the chromatography specimen is adherently covered with the liquid-impermeable sheet material.

5. The chromatography measuring device as defined in any of Claims 1 to 4, wherein
the chromatography specimen is constituted by laminating or connecting plural porous materials.

6. The chromatography measuring device as defined in any of Claims 1 to 4, wherein '
the chromatography specimen is composed of a single-layer porous material.

7. The chromatography measuring device as defined in Claim 6, wherein
the single-layer porous material is nitrocellulose.

8. The chromatography measuring device as defined in any of Claims 1 to 7, wherein the chromatographic downstream region which is not covered with the liquid-impermeable sheet material is covered with a gas-permeable material.

9. The chromatography measuring device as defined in Claim 8, wherein
the gas-permeable material is an arbitrary porous thin-film material such as a nonwoven fabric.

10. The chromatography measuring device as defined in Claim 8, wherein
the gas-permeable material is retiform tissue.

11. The chromatography measuring device as defined in any of Claims 1 to 7, wherein
a space forming part for forming arbitrary space is provided on the chromatographic downstream region which is not covered with the liquid-impermeable sheet material.

12. The chromatography measuring device as defined in Claim 11, wherein
a gap part is provided in an arbitrary region, such as at the end or on a parallel side of the chromatographic downstream region in the space forming part, or on the top surface of the space forming part, so as to enable air inflow.

13. The chromatography measuring device as defined in Claim 11 or 12, wherein
the space forming part is composed of a liquid-impermeable material.

14. The chromatography measuring device as defined in any of Claims 1 to 13, wherein
the chromatography specimen is an immunochromatography specimen employing an antigen-antibody reaction.

15. The chromatography measuring device as defined in any of Claims 1 to 14, wherein
the chromatography specimen is a dry analysis element.

16. The chromatography measuring device as defined in any of Claims 1 to 15, wherein
the chromatography specimen is a one-step specimen.

## Patentansprüche

1. Chromatographisches Messgerät, das eine chromatographische Probe als Probe zum Durchführen einer chromatographischen Messung hat und eine zu prüfende Substanz, die auf die chromatographische Probe aufgebracht wird, qualitativ oder quantitativ misst,
**dadurch gekennzeichnet, dass** die chromatographische Probe mindestens an der Oberseite und den Seitenflächen von beiden Endbereichen an einem hinteren und einem vorderen Chromatographie-Ende offen ist und an allen Stellen der Oberseite außer ihren beiden Endbereichen an dem hinteren und vorderen Chromatographie-Ende mit einem flüssigkeitsundurchlässigen Folienmaterial haftend bedeckt ist.

2. Chromatographisches Messgerät nach Anspruch 1, **dadurch gekennzeichnet, dass** die chromatographische Probe weiterhin an allen Stellen der Seitenflächen außer ihren beiden Endbereichen an dem hinteren und vorderen Chromatographie-Ende mit einem flüssigkeitsundurchlässigen Folienmaterial haftend bedeckt ist.

3. Chromatographisches Messgerät nach Anspruch 1, **dadurch gekennzeichnet, dass** die chromatographische Probe weiterhin an allen Stellen der Seitenflächen und der Unterseite außer ihren beiden Endbereichen an dem hinteren und vorderen Chromatographie-Ende mit einem flüssigkeitsundurchlässigen Folienmaterial haftend bedeckt ist.

4. Chromatographisches Messgerät nach einem der Ansprüche 1 bis 3, **dadurch gekennzeichnet, dass** ein Messbereich mindestens von einem am hinteren Ende befindlichen Markerreagens-Halteteil, in dem ein Markerreagens gehalten wird, bis zu einem am vorderen Ende befindlichen Spezifisches-Protein-Immobilisierungsteil, in dem ein spezifisches Protein immobilisiert wird, an der chromatographischen Probe mit dem flüssigkeitsundurchlässigen Folienmaterial haftend bedeckt ist.

5. Chromatographisches Messgerät nach einem der Ansprüche 1 bis 4, **dadurch gekennzeichnet, dass** die chromatographische Probe durch Aufeinanderschichten oder Verbinden mehrerer poröser Materialien hergestellt wird.

6. Chromatographisches Messgerät nach einem der Ansprüche 1 bis 4, **dadurch gekennzeichnet, dass** die chromatographische Probe aus einem einschichtigen porösen Material besteht.

7. Chromatographisches Messgerät nach Anspruch 6, **dadurch gekennzeichnet, dass** das einschichtige poröse Material Cellulosenitrat ist.

8. Chromatographisches Messgerät nach einem der Ansprüche 1 bis 7, **dadurch gekennzeichnet, dass** der chromatographische Bereich am vorderen Ende, der nicht mit dem flüssigkeitsundurchlässigen Folienmaterial bedeckt ist, mit einem gasdurchlässigen Material bedeckt ist.

9. Chromatographisches Messgerät nach Anspruch 8, **dadurch gekennzeichnet, dass** das gasdurchlässige Material ein beliebiges poröses Dünnschichtmaterial, wie etwa ein Vliesstoff, ist.

10. Chromatographisches Messgerät nach Anspruch 8, **dadurch gekennzeichnet, dass** das gasdurchlässige Material Netzgewebe ist.

11. Chromatographisches Messgerät nach einem der Ansprüche 1 bis 7, **dadurch gekennzeichnet, dass** ein Raumbildungsteil zum Bilden eines beliebigen Raums in dem vorderen chromatographischen Bereich, der nicht mit dem flüssigkeitsundurchlässigen Folienmaterial bedeckt ist, vorgesehen ist.

12. Chromatographisches Messgerät nach Anspruch 11, **dadurch gekennzeichnet, dass** ein Spaltteil in einem beliebigen Bereich, wie etwa am Ende oder an einer parallelen Seite des vorderen chromatographischen Bereichs in dem Raumbildungsteil oder an der Oberseite des Raumbildungsteils, vorgesehen ist, um ein Einströmen von Luft zu ermöglichen.

13. Chromatographisches Messgerät nach Anspruch 11 oder 12, **dadurch gekennzeichnet, dass** der Raumbildungsteil aus einem flüssigkeitsundurchlässigen Material besteht.

14. Chromatographisches Messgerät nach einem der Ansprüche 1 bis 13, **dadurch gekennzeichnet, dass** die chromatographische Probe eine immunchromatographische Probe ist, die eine Antigen-Antikörper-Reaktion verwendet.

15. Chromatographisches Messgerät nach einem der Ansprüche 1 bis 14, **dadurch gekennzeichnet, dass** die chromatographische Probe ein Trockenanalyse-Element ist.

16. Chromatographisches Messgerät nach einem der Ansprüche 1 bis 15, **dadurch gekennzeichnet, dass** die chromatographische Probe eine Einstufenprobe ist.

## Revendications

1. Dispositif de mesure par chromatographie qui a un spécimen chromatographique en tant que spécimen pour effectuer une mesure chromatographique et mesure qualitativement ou quantitativement une substance à tester, qui est appliquée au spécimen chromatographique, dans lequel
le spécimen chromatographique est ouvert sur au moins les surfaces supérieure et latérale des deux régions d'extrémités sur l'amont et l'aval chromatographique, et est recouvert avec adhérence par un matériau en feuille imperméable au liquide dans toutes les parties de la surface supérieure à l'exception de ses deux régions d'extrémités sur l'amont et l'aval chromatographique.

2. Dispositif de mesure par chromatographie comme défini dans la revendication 1, dans lequel
le spécimen chromatographique est en plus recouvert avec adhérence par un matériau en feuille imperméable au liquide dans toutes les parties des surfaces latérales à l'exception de ses deux régions d'extrémités sur l'amont et l'aval chromatographique.

3. Dispositif de mesure par chromatographie comme défini dans la revendication 1, dans lequel
le spécimen chromatographique est en plus recouvert avec adhérence par un matériau en feuille imperméable au liquide dans toutes les parties des surfaces latérales et de la surface inférieure à l'exception de ses deux régions d'extrémités sur l'amont et l'aval chromatographique.

4. Dispositif de mesure par chromatographie comme défini dans l'une quelconque des revendications 1 à 3, dans lequel
une région de mesure au moins d'une partie de maintien de réactif marqueur où un réactif marqueur est maintenu, située en amont, à une partie d'immobilisation d'une protéine spécifique, située en aval, dans le spécimen chromatographique est recouverte avec adhérence par le matériau en feuille imperméable au liquide.

5. Dispositif de mesure par chromatographie comme défini dans l'une quelconque des revendications 1 à 4, dans lequel
le spécimen chromatographique est constitué par la stratification ou la liaison de plusieurs matériaux poreux.

6. Dispositif de mesure par chromatographie comme défini dans l'une quelconque des revendications 1 à 4, dans lequel
le spécimen chromatographique est composé d'un matériau poreux à une seule couche.

7. Dispositif de mesure par chromatographie comme défini dans la revendication 6, dans lequel
le matériau poreux à une seule couche est de la nitrocellulose.

8. Dispositif de mesure par chromatographie comme défini dans l'une quelconque des revendications 1 à 7, dans lequel
la région en aval chromatographique qui n'est pas recouverte par le matériau en feuille imperméable au liquide est recouverte par un matériau perméable au gaz.

9. Dispositif de mesure par chromatographie comme défini dans la revendication 8, dans lequel
le matériau perméable au gaz est un matériau à film mince poreux arbitraire tel qu'un tissu non tissé.

10. Dispositif de mesure par chromatographie comme défini dans la revendication 8, dans lequel
le matériau perméable au gaz est un tissu rétiforme.

11. Dispositif de mesure par chromatographie comme défini dans l'une quelconque des revendications 1 à 7, dans lequel
une partie formant un espace destinée à former un espace arbitraire est procurée sur la région en aval chromatographique qui n'est pas recouverte par le matériau en feuille imperméable au liquide.

12. Dispositif de mesure par chromatographie comme défini dans la revendication 11, dans lequel
une partie d'écartement est pourvue dans une région arbitraire, telle qu'à l'extrémité ou sur un côté parallèle de la région en aval chromatographique dans la partie formant un espace, ou sur la surface supérieure de la partie formant un espace, de façon à permettre l'influx d'air.

13. Dispositif de mesure par chromatographie comme défini dans la revendication 11 ou 12, dans lequel
la partie formant un espace est composée d'un matériau imperméable au liquide.

14. Dispositif de mesure par chromatographie comme défini dans l'une quelconque des revendications 1 à 13, dans lequel
le spécimen chromatographique est un spécimen immunochromatographique employant une réaction antigène-anticorps.

15. Dispositif de mesure par chromatographie comme défini dans l'une quelconque des revendications 1 à 14, dans lequel
le spécimen chromatographique est un élément d'analyse sec.

16. Dispositif de mesure par chromatographie comme défini dans l'une quelconque des revendications 1 à 15, dans lequel
le spécimen chromatographique est un spécimen à une étape.
